# EUROPEAN PATENT SPECIFICATION

(11) **EP 0 398 945 B1**
(45) Date of publication and mention of the grant of the patent: **12.05.1993**
(21) Application number: 89902029.1
(22) Date of filing: 18.01.1989
(51) Int. Cl.: A61K 7/06, A61K 31/44

(54) **NEW TOPICAL PREPARATION FOR TREATMENT OF ALOPECIA**
TOPISCHE ZUBEREITUNG ZUR BEHANDLUNG VON ALOPECIE
NOUVELLE PREPARATION TOPIQUE SERVANT AU TRAITEMENT DE L'ALOPECIE

(30) Priority: 21.01.1988 GB 8801318
(43) Date of publication of application: 28.11.1990
(73) Proprietor: LEO PHARMACEUTICAL PRODUCTS LTD. A/S (LOVENS KEMISKE FABRIK PRODUKTIONSAKTIESELSKAB), 2750 Ballerup (DK)
(72) Inventor: GODTFREDSEN, Wagn, Ole, DK-3500 Värlose (DK)
(74) Representative: Kinzebach, Werner, Dr.
(86) International application number: DK8900006
(87) International publication number: WO8906530

(56) References cited:
- EP-A- 0 207 606
- EP-A- 0 207 606
- EP-A- 0 207 606
- EP-A- 0 249 397
- EP-A- 0 260 790
- EP-A- 0 260 790
- EP-A- 0 260 790
- WO-A-85/04577
- WO-A-88/04171
- WO-A-60/6960
- US-A- 4 057 636
- US-E- 31 244

## Description

This invention relates to the use of pyridylguanidine compounds for the manufacture of a composition for increasing the rate of terminal hair growth and/or stimulating the conversion of vellus hair to terminal hair and the growth thereof.

The compounds used according to the present invention are known as hypotensives e.g. described in US-A-4 057 636 and in the corresponding US-patent Re. 31 244. One of said compounds is pinacidil which is N''-cyano-N-4-pyridinyl-N''-(1,2,2-trimethylpropyl) guanidine. EP-A-207 606 discloses that pinacidil is also a potent bronchodilating agent useful in the treatment of asthma in mammals and WO-A-86/06960 discloses the use of pinacidil for the treatment of peripheral vascular disease.

Said compounds have the general formula I: 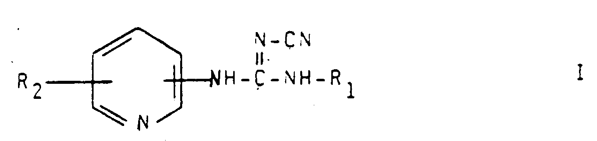
in which the R₁- substituted cyano-guanidyl radical is placed in the 3- or 4-position of the pyridine ring, and in which R₁ stands for a straight or branched, saturated or unsaturated, aliphatic hydrocarbon radical having from 1 to 8 carbon atoms, a cycloalkyl or cycloalkenyl radical having from 3 to 7 carbon atoms, or an aryl or an aralkyl radical. R₂ stands for hydrogen, halogen, hydroxy, lower alkyl or alkoxy radicals. The present invention comprises all stereoisomers of formula I as well as mixtures thereof.

In particular, the present composition and process use as active compound of formula I either N-tert-butyl-N''-cyano-N'-3-pyridylguanidine (in the following also designated P 1060 or N''-cyano-N-4-pyridyl-N'-1,2,2-trimethylpropylguanidine (in the following also designated pinacidil).

Alopecia (a partial or complete loss of hair) may result from genetic factors, aging, or from local or sysstemic disease. Male-pattern baldness is extremely common. It is familial and requires the presence of androgens, but other etiological factors are unknown. Female-pattern alopecia is not infrequent in women. It is ordinarily confined to thinning of hair in the frontal and the parietal regions. Complete baldness in any area is rare.

Alopecia is due to a deficiency of terminal hair which is the visual coloured hair. If the hair loss is due to atrophy or scarring, no regrowth can be expected. But in other cases, even where there is a noticeable absence of terminal hair, the skin of a seemingly bald person may contain the socalled vellus hair which is a very fine colourless hair, the presence of which needs microscopic determination. The vellus hair is a precursor to the terminal hair, and a regrowth may thus be promoted both by influencing the conversion of vellus hair to terminal hair and by stimulating the growth of the latter.

As alopecia is mainly a cosmetic problem, any therapy should never present risks which are unjustifiable. It has surprisingly turned out that the compounds of formula I can be effectively used for the desired purpose. They find applications both in the human and veterinary use, the latter in particular being of economic importance in connection with animals raised for their pelts, e.g. mink. The present compositions can be used over the entire surface of the body for improving the pelt for commercial reasons, or they can be used partially to cure e.g. bald patches.

The pharmaceutical compositions contemplated by this invention include pharmaceutical compositions suited for topical application.

The term "topical" as employed herein relates to the use of a compound of the formula I, incorporated in a suitable pharmaceutical topical base in form of a solution and/or a suspension in which the active compound is suspended as a microfine powder. Accordingly, such topical compositions include those pharmaceutical forms in which the compound is applied externally by direct contact with the skin surface to be treated. Conventional pharmaceutical forms for this purpose include ointments, lotions, pastes, jellies, sprays or aerosols. The term "ointment" embraces formulations (including creams) having oleaginous, absorption, water-soluble and emulsion-type bases, e.g. petrolatum, lanolin, polyethylene glycols, as well as mixtures of these.

The present compositions may advantageously further contain percutaneous penetration enhancers, such as N-methyl-2-pyrrolidone, azone, propylene glycol, and poly(oxyethylene)-poly(oxypropylene) co-polymers.

The percentage by weight of the compound of the formula I herein utilized ranges from 0.1% to 20.0% of the pharmaceutical preparation, preferably from 0.5% to 5 % and in these preparations the aforesaid pharmaceutical carrier for topical application constitutes a major amount of the said preparation.

The following examples describe the manner and process of making and using the invention and set forth the best mode contemplated by the inventor of carrying out the invention but are not to be construed as limiting.

### Example 1

| Topical ointment | |
|---|---|
| P 1060 | 20 g |
| Paraffin, liquid | 200 g |
| White soft paraffin to make | 1000 g |

The fatty ingredients are melted at increased temperature, and the active substance P 1060 is incorporated by homogenizing. The ointment is cooled and filled into suitable containers.

### Example 2

| Topical ointment | |
|---|---|
| Pinacidil | 100 g |
| Sorbitan sesquioleate | 50 g |
| Paraffin, liquid | 100 g |
| White soft paraffin to make | 1000 g |

Preparation as described in Example 1.

### Example 3

| Topical (waterfree/watermiscible) ointment | |
|---|---|
| P 1060 | 50 g |
| Polyethylene glycol 400 | 250 g |
| Polyethylene glycol 4000 | 700 g |

The glycols are melted at increased temperature. The active substance P 1060 is incorporated. The preparation is cooled and filled into suitable containers.

### Example 4

| Topical cream | |
|---|---|
| Pinacidil | 50 g |
| Paraffin, liquid | 100 g |
| White soft paraffin | 50 g |
| Cetyl alcohol | 100 g |
| Polyoxyethylene sorbitan monostearate | 50 g |
| Methylparaben | 2 g |
| Propylparaben | 0.2 g |
| Glycerol | 100 g |
| Water to make | 1000 g |

The fatty ingredients including the emulsifying agent are melted by increased temperature. At increased temperature the water phase including the active substance pinacidil and a solution of the preservative are mixed with the melted fatty phase. The cream is homogenized, cooled, and filled into suitable containers.

### Example 5

| Topical cream | |
|---|---|
| P 1060 | 10 g |
| Cetostearyl alcohol | 100 g |
| White soft paraffin | 150 g |
| Liquid paraffin | 50 g |
| Cetomacrogal 1000 | 20 g |
| Chlorocresol | 1 g |
| Water to make | 1000 g |

Preparation as described in Example 4.

### Example 6

| Topical hydrogel | |
|---|---|
| Pinacidil | 50 g |
| Carbomer | 10 g |
| Methylparaben | 2 g |
| Propylparaben | 0.2 g |
| Silicone oil | 30 g |
| Triethanolamine | 5 g |
| Water to make | 1000 g |

The preservatives are dissolved and mixed with the carbomer. A gel is formed by the addition of triethanolamine. Finally, pinacidil and silicone oil are added, and the gel is homogenized. The gel is filled into suitable containers.

### Example 7

| Topical gel | |
|---|---|
| P 1060 | 10 g |
| Liquid paraffin | 20 g |
| Cetostearyl alcohol | 20 g |
| Polyoxyethylene-2-stearylether | 3 g |
| Polyoxyethylene-10-stearylether | 7 g |
| Methylparaben | 2 g |
| Propylparaben | 0.2 g |
| Propylene glycol | 100 g |
| Carbomer | 10 g |
| Triethanolamine | 5 g |
| Water to make | 1000 g |

The fatty ingredients including the emulsifying agent are melted at increased temperature. The water phase including a solution and/or a homogeneous suspension of the active substance P 1060, the preservatives, and the carbomer are mixed with the fatty phase.

The mixture is homogenized, gelled by the addition of the triethanolamine, and then filled into suitable containers.

### Example 8

| Topical lotion | |
|---|---|
| P 1060 | 30 g |
| Propylene glycol | 50 g |
| Isopropyl alcohol | 850 g |
| Water to make | 1000 g |

The active substance is dissolved in the pharmaceutical base.

### Example 9

| Topical lotion | |
|---|---|
| Pinacidil | 50 g |
| Polyethylene glycol 4000 | 120 g |
| Myristyl-γ-picolinium chloride | 0.2 g |
| Polyvinylpyrrolidone | 1 g |
| Deionized water q.s. ad | 1000 c.c. |

The ingredients are dissolved in water and filled into containers.

The composition so prepared can be used in the topical treatment of baldness by application to the scalp daily.

### Example 10

| Topical spray | |
|---|---|
| Aerosol (foam) | |
| P 1060 | 10 g |
| Polawax A 31 | 40 g |
| Ethyl alcohol | 600 g |
| Polyvinylpyrrolidone | 30 g |
| Glycerol | 10 g |
| Water | 220 g |
| Dichlorodifluoromethan | 40 g |
| Dichlorotetrafluoroethan | 60 g |

All ingredients are dissolved or suspended in ethyl alcohol and the water. The concentrate is filled into aerosol containers and the propellants are added.

### Example 11

| Topical Spray | |
|---|---|
| Aerosol | |
| Pinacidil | 1.5 g |
| Absolute alcohol | 4.3 g |
| Dichlorodifluoroethane | 1.43 g |
| Dichlorotetrafluoroethane | 5.70 g |

The pinacidil is suspended in the absolute alcohol. The suspension is chilled to about minus 30°C. To this is added the chilled mixture of dichlorodifluoromethane and dichlorotetrafluoroethane. 13 ml plastic-coated amber bottles are cold filled with 11.5 g each of the resulting solution and capped.

The compositions can be sprayed on the scalp daily to convert vellus hair to growth as terminal hair.

### Example 12

| Dusting Powder | |
|---|---|
| P 1060 | 10 g |
| Magnesium stearate | 5 g |
| Silicone dioxide colloidal | 10 g |
| Lactose | 478 g |
| Maize starch | 500 g |

The powdered ingredients are mixed together.

### Example 13

| Dusting Powder | |
|---|---|
| P 1060 | 10 g |
| Bentonite | 100 g |
| Talc q.s. | 1000 g |

The powdered ingredients are mixed together and dusted on the fur of minks for increasing the rate of hair growth.

## Claims

1. The use of a compound of formula I 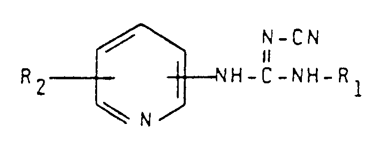 in which the R₁-substituted cyano-guanidyl radical is placed in the 3- or 4-position of the pyridine ring, and in which R₁ stands for a straight or branched, saturated or unsaturated, aliphatic hydrocarbon radical having from 1 to 8 carbon atoms, a cycloalkyl or cycloalkenyl radical having from 3 to 7 carbon atoms, or an aryl or an aralkyl radical, and R₂ stands for hydrogen, halogen, hydroxy, lower alkyl or alkoxy radicals; all stereoisomers thereof and mixtures of the same, and the pharmaceutically acceptable acid addition salts thereof for the manufacture of a composition for increasing the rate of terminal hair growth and/or stimulating the conversion of vellus hair to terminal hair and the growth thereof.

2. The use according to claim 1 wherein the compound is N-tert-butyl-N''-cyano-N'-3-pyridylguanidine or an acid addition salt thereof.

3. The use according to claim 1 wherein the compound is N''-cyano-N-4-pyridyl-N'-1,2,2-trimethylpropylguanidine or an acid addition salt thereof.

## Patentansprüche

1. Verwendung einer Verbindung der Formel I 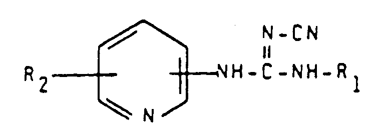 worin der R₁-substituierte Cyanoguanidylrest in 3- oder 4-Position des Pyridinrings steht und R₁ für einen geradkettigen oder verzweigten, gesättigten oder ungesättigten, aliphatischen Kohlenwasserstoffrest mit 1 bis 8 Kohlenstoffatomen, einen Cycloalkyl- oder Cycloalkenylrest mit 3 - 7 Kohlenstoffatomen oder einen Aryl- oder Aralkylrest steht und R₂ für ein Wasserstoffatom, Halogenatom, einen Hydroxy-, Niedrigalkyl- oder-alkoxyrest steht, aller Stereoisomeren davon und der Mischungen davon sowie der pharmazeutisch verträglichen Säureadditionssalze davon zur Herstellung eines Mittels zur Erhöhung der Wachstumsrate von Terminalhaar und/oder zur Stimulierung der Umwandlung von Vellushaar in Terminalhaar und des Wachstums davon.

2. Verwendung nach Anspruch 1, wobei es sich bei der Verbindung um N-tert-Butyl-N''-cyano-N'-3-pyridylguanidin oder ein Säureadditionssalz davon handelt.

3. Verwendung nach Anspruch 1, wobei es sich bei der Verbindung um N''-Cyano-N-4-pyridyl-N'-1,2,2-trimethylpropylguanidin oder ein Säureadditionssalz davon handelt.

## Revendications

1. Utilisation d'un composé de formule I : 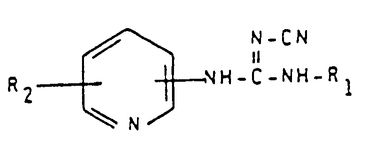 dans laquelle le radical cyano-guanidyle R₁-substitué est en position 3 ou 4 du noyau pyridine et dans laquelle R₁ représente un radical hydrocarboné aliphatique, saturé ou insaturé, à chaîne droite ou ramifiée en C₁-C₈, un radical cycloalkyle ou cycloalcényle en C₃-C₇ ou un radical aryle ou aralkyle et R₂ représente l'hydrogène ou un halogène ou un groupe hydroxy, alkyle inférieur ou alcoxy inférieur; de tous ses stéréoisomères et leurs mélanges et de leurs sels d'addition d'acides pharmaceutiquement acceptables pour la fabrication d'une composition pour augmenter la vitesse de croissance du cheveu terminal et/ou stimuler la conversion du cheveu de duvet en cheveu terminal et sa croissance.

2. Utilisation selon la revendication 1, dans laquelle le composé est la N-(tert-butyl)-N''-cyano-N'-(3-pyridyl)-guanidine ou un de ses sels d'addition d'acides.

3. Utilisation selon la revendication 1, dans laquelle le composé est la N''-cyano-N-(4-pyridyl)-N'-(1,2,2-triméthylpropyl)-guanidine ou un de ses sels d'addition d'acides.
